# EUROPÄISCHE PATENTSCHRIFT

(11) **EP 2 621 892 B1**
(45) Veröffentlichungstag und Bekanntmachung des Hinweises auf die Patenterteilung: **29.07.2015**
(21) Anmeldenummer: 11758206.4
(22) Anmeldetag: 21.09.2011
(51) Int. Cl.: C07C 227/42, B01J 2/06, B01J 8/18, C11D 3/33, C11D 11/00, B01J 19/26

(54) **VERFAHREN ZUR HERSTELLUNG EINES GRANULATES ENTHALTEND EINES ODER MEHRERE KOMPLEXBILDNERSALZE**
PROCESS FOR PRODUCING GRANULES COMPRISING ONE OR MORE COMPLEXING AGENT SALTS
PROCÉDÉ POUR PRODUIRE DES GRANULÉS CONTENANT UN OU PLUSIEURS SELS COMPLEXANTS

(30) Priorität: 27.09.2010 EP 10180031
(43) Veröffentlichungstag der Anmeldung: 07.08.2013
(73) Patentinhaber: BASF SE, 67056 Ludwigshafen (DE)
(72) Erfinder: BLEI, Stefan, 68199 Mannheim (DE); SCHÖNHERR, Michael, 67227 Frankenthal (DE); WEBER, Franz, 69214 Eppelheim (DE); BECKER, Francois, 76835 Burrweiler (DE)
(86) Internationale Anmeldenummer: PCT/EP2011/066376
(87) Internationale Veröffentlichungsnummer: WO 2012/041741

(56) Entgegenhaltungen:
- EP-A2- 0 845 456
- WO-A1-2009/103822
- WO-A2-2011/023382
- D. GEHRMANN: "Trockner", CHEMIE INGENIEUR TECHNIK, Bd. 78, Nr. 12, 1. Dezember 2006 (2006-12-01), Seiten 1778-1789, XP55015072, ISSN: 0009-286X, DOI: 10.1002/cite.200690141

## Beschreibung

Die Erfindung betrifft ein Verfahren zur Herstellung eines Granulats enthaltend eines oder mehrere Komplexbildnersalze der allgemeinen Formel I 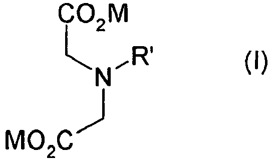 sowie eine Verwendung der obigen Granulate.

Die häufig als Komplexbildner beispielsweise in Wasch- und Reinigungsmitteln eingesetzten Aminopolyphosphonate, Polycarboxylate oder Aminopolycarboxylate, wie Ethylendiamintetraessigsäure (EDTA), sind nur in geringem Maße biologisch abbaubar.

Eine preiswerte Alternative stellen die Glycin-N,N-diessigsäurederivate, wie die Methylglycin-N,N-diessigsäure (MGDA) und deren Salze - z.B. die Trialkalimetallsalze - dar, welche vorteilhafte toxikologische Eigenschaften aufweisen und gut biologisch abbaubar sind. Die Verwendung von MGDA und von verwandten Glycin-N,N-diessigsäurederivaten in Reinigungsmitteln sowie deren Synthesen sind z.B. in WO-A 94/029421 oder US 5,849,950 beschrieben. Für eine kostengünstige Produktion der Glycin-N,N-diessigsäurederivate werden hohe Anforderungen an die Ausbeute der einzelnen Syntheseschritte und Reinheit der isolierten Zwischenprodukte gestellt.

MGDA wird insbesondere durch Umsetzung von Iminodiacetontril mit Acetaldehyd und Blausäure oder von alpha-Alaninnitril mit Formaldehyd und Blausäure und alkalischer Hydrolyse des als Zwischenprodukt erhaltenen Methylglycindiacetontrils (MGDN) mit Natronlauge hergestellt, wobei das Trinatriumsalz des MGDA erhalten wird. Um hohe MGDA-Ausbeuten und -Reinheiten zu erzielen, wird MGDN in der Regel als Zwischenprodukt isoliert und als Reinstoff in dem sich anschließenden Hydrolyseschritt eingesetzt.

Problematisch bei der Hydrolyse von Alkylglycinnitril-N,N-diacetonitrilen ist ihre thermische Labilität, insbesondere im alkalischen Milieu. Durch die sterisch anspruchsvolle Alkvlsubstitution werden Rückspaltungsreaktionen begünstigt. Deshalb sind Verfahren entwickelt worden, die möglichst zu nebenproduktarmen Formen des MGDA und seiner Salze führen.

Ein verbessertes Verfahren zur Herstellung von nebenproduktarmen Salzen des MGDA wird in der WO 2006/120129 beschrieben. Die moderneren Herstellverfahren führen im Allgemeinen zu etwa 35 bis 40 gew.-%igen wässrigen Lösungen, aus denen danach die Salze in fließfähiger Form hergestellt werden.

Eines der bekannten Aufarbeitungsverfahren im Stand der Technik ist die Überführung derartiger wässriger Lösungen in einen Sprühturm. Dabei entstehen überwiegend amorphe Pulver mit einer Restfeuchte in der Größenordnung von beispielsweise 5 Gew.-%. Höhere Restfeuchten sind zwar denkbar, aber in einem Sprühturm eher schwierig zu erzeugen und darüber hinaus auch unerwünscht, weil es dann bei der späteren Lagerung beim Abnehmer oder bei der Verarbeitung Verklumpungen der Pulver geben kann. Es ist weiterhin bekannt, dass ein Granulat solche Nachteile nicht hat und sich deshalb problemloser verarbeiten lässt. Allerdings verlangt die Granulaterzeugung einen zusätzlichen Umarbeitungsschritt nach der Pulvererzeugung im Sprühturm und ist deshalb relativ teuer. Bei diesem Umarbeitungsschritt wird dem Pulver aus dem Sprühturm zusätzliche Feuchtigkeit zugeführt, und es wird unter Erwärmen und Kneten bei einer Verweilzeit in der Größenordnung einer Stunde über eine Kristallisation granuliert. Ein derartiges Verfahren ist beispielsweise in EP-A 08 45 456 beschrieben.

Komplexbildnersalze sind häufig in grobkörniger Form erwünscht, weil diese mit vielfachen Vorteilen verbunden ist, insbesondere mit einem verbesserten Fließverhalten, einer leichteren Handhabung und einer verbesserten Dosierbarkeit.

Ein besonders vorteilhaftes Verfahren zur Herstellung von grobkörnigen Produkten ist die Granulation in einer speziellen Wirbelschicht, der so genannten Strahlschicht, auch als Wirbelschicht-Sprühgranulation bekannt. Das Verfahren ist beispielsweise Gegenstand der Diplomarbeit von A. Werner: Wirbelschicht-Sprühgranulation: Prozessoptimierung in der Strahlschicht, September 2009, BASF SE und Universität Stuttgart, Institut für mechanische Verfahrenstechnik.

Die entsprechenden Apparate sind in der Fach- und Patentliteratur häufig als Strahlschichtapparate bezeichnet und beispielsweise in DE 10 2005 037 630, DE 10 162 781 oder DE 103 22 062, beschrieben.

In diesen Apparaten bildet sich jedoch, anders als bei einer klassischen Wirbelschicht, keine deutliche sichtbare Grenzschicht zwischen dem Wirbelgut und dem darüber liegenden Gasraum aus, so dass vorliegend hierfür der Begriff Strahlapparat verwendet wird.

Die WO 2009/103822 beschreibt ein Verfahren zur Herstellung von freifließenden Granulaten eines oder mehrerer Salze der Methylglycindiessigsäure (MGDA) mit niedriger Hygroskopizität, wobei eine konzentrierte Lösung mit einem Feststoffgehalt im Bereich zwischen 45 und 70 % und einer Temperatur zwischen 50 und 120 °C, bevorzugt bei 80 °C, erwärmt und anschließend einer Sprühgranulation durch Einleiten eines Luftstromes mit einer Temperatur von 120 °C oder weniger unterworfen wird (vergleiche D1, Anspruch 1).

Der Fachartikel von D. Gehrmann: "Trockner" aus Chemie Ingenieur Technik, Band 78, Nr. 12, Seite 1778-1789, gibt eine Übersicht über die unterschiedlichen Trocknertypen. Auf Seite 1780, 3. Spalte wird auf den ProCell-Apparat, einen kontinuierlichen Strahlschichttrockner hingewiesen, als im Mittelpunkt der Neuentwicklung stehend. Der Apparat weist einen durch zwei bewegliche Walzen verstellbaren Luftspalt auf, mit dem gezielt auch ein zum Verkleben neigendes Produkt fluidisiert werden kann.

Aufgabe der Erfindung war es, ein Verfahren zur Herstellung von Granulaten eines oder mehrerer Komplexbildnersalze mit verbesserter Raum-Zeit-Ausbeute und verbesserer Produktqualität, insbesondere mit kompakterer, gleichmäßigerer Partikelform und mit hiervon abgeleiteten Größen, insbesondere höherer Schüttdichte, niedrigerer Bruchempfindlichkeit und besserer Fließfähigkeit, zur Verfügung zu stellen.

Die Aufgabe wird gelöst durch ein Verfahren zur Herstellung eines Granulates enthaltend eines oder mehrere Komplexbildnersalze der allgemeinen Formel 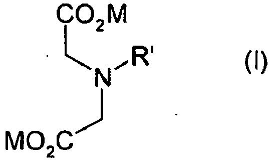 in der
- R': Wasserstoff oder eine der Gruppierungen 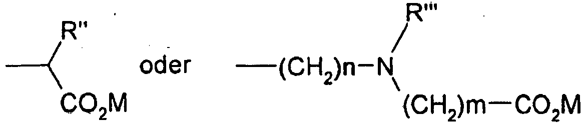 bedeutet, wobei
- R'': Wasserstoff, ein C₁-C₁₂-Alkylrest oder ein -(CH₂)_{q}-COOM-Rest mit q = 1 bis 5 ist
- n und m: jeweils eine ganze Zahl von 0 bis 5 sind und
- R''': Wasserstoff oder ein C₁-C₁₂-Alkylrest oder ein C₂-C₁₂-Alkenylrest ist, der zusätzlich mit bis zu 5 Hydroxylgruppen substituiert sein kann, oder eine der Gruppierungen 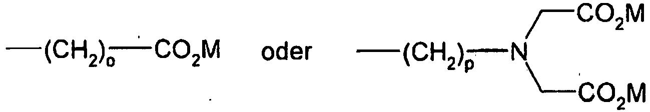
ist, in der o und p jeweils eine ganze Zahl von 0 bis 5 sind, und M unabhängig voneinander Wasserstoff, Alkalimetall, Erdalkalimetall, Ammonium oder substituiertes Ammonium in den entsprechenden stöchiometrischen Mengen bedeutet,
- aus einer wässrigen Ausgangslösung, enthaltend das eine oder die mehreren Komplexbildnersalze in einer Konzentration von 10 bis 80 Gew.-%, bezogen auf das Gesamtgewicht der wässrigen Ausgangslösung,
in einem Strahlapparat, in dem ein von unten nach oben gerichteter, zentral oder ein oder mehrere, im Bereich der Mittelachse des Strahlapparates positionierte gasförmige Treibstrahle eine interne Schlaufenbewegung induzieren, unter Ausbildung einer Strahlzone, an deren oberen Ende sich eine Fontänenzone anschließt, die in eine Rücklaufzone im Wandbereich des Strahlapparates übergeht, die erneut, in ihrem unteren Bereich, in die Strahlzone übergeht, wo die wässrige Ausgangslösung in den einen oder die mehreren gasförmigen Treibstrahle eingedüst wird und dabei unter Erhalt des Granulates getrocknet wird, das aus dem Strahlapparat ausgetragen wird.

Durch das definierte Strömungsprofil im Strahlapparat, mit interner Schlaufenbewegung, wird bewirkt, dass die sich bildenden Granulatkörner regelmäßig an der einen oder den mehreren Düsen vorbeikommen, über die die wässrige Ausgangslösung eingesprüht wird, so dass regelmäßig, in deutlich definierten Zeitabständen besprüht werden, mit der Folge, dass es zum regelmäßigen Aufwachsen von übereinander liegenden Schichten, in der Art von übereinander liegenden Zwiebelschalen kommt, und somit zu sehr gleichförmigen Partikeln aufwachsen. Die hierbei erhaltenen Granulate weisen ausgezeichnete Produkteigenschaften auf, insbesondere sehr hohe, definierte Schüttdichte, im Bereich zwischen 650 und 1000 kg/m³, insbesondere zwischen 760 und 920 kg/m³, sowie eine definierte, für den Einsatzzweck geforderte Restfeuchte, im Bereich von ca. 6 bis 14 Gew.-% Wasser, insbesondere 11 bis 13 Gew.-% Wasser.

Granulate der obigen Komplexbildnersalze mit einer bevorzugten Restfeuchte von > 8 Gew.-%, insbesondere > 12 Gew.-%, verkleben häufig in Standard-Wirbelschichtapparaten, können jedoch problemlos in den erfindungsgemäß eingesetzten Strahlapparaten hergestellt werden.

Hierzu wird von einer wässrigen Lösung eines oder mehrerer Komplexbildnersalze ausgegangen, mit einer Konzentration im Bereich von 10 bis 80 Gew.-%, bezogen auf das Gesamtgewicht der wässrigen Lösung. Die wässrige Ausgangslösung kann bevorzugt bis unterhalb des Siedepunkts derselben vorerwärmt werden.

Das eine oder die mehreren Komplexbildnersalze entsprechen der allgemeinen Formel 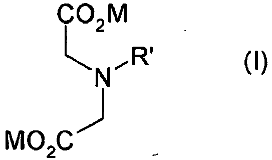 in der
R' Wasserstoff oder eine der Gruppierungen 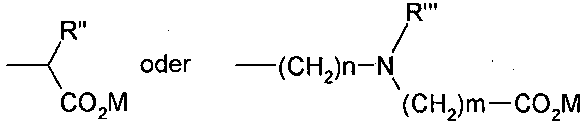 bedeutet, wobei
- R'': Wasserstoff, ein C₁-C₁₂-Alkylrest oder ein -(CH₂)_{q}-COOM-Rest mit q = 1 bis 5 ist
- n und m: jeweils eine ganze Zahl von 0 bis 5 sind und
- R''': Wasserstoff oder ein C₁-C₁₂-Alkylrest oder ein C₂-C₁₂-Alkenylrest ist, der zusätzlich mit bis zu 5 Hydroxylgruppen substituiert sein kann, oder eine der Gruppierungen 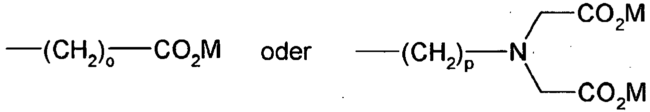
ist, in der o und p jeweils eine ganze Zahl von 0 bis 5 sind, und
M unabhängig voneinander Wasserstoff, Alkalimetall, Erdalkalimetall, Ammonium oder substituiertes Ammonium in den entsprechenden stöchiometrischen Mengen bedeutet.

Bevorzugt handelt es sich hierbei um Derivate der Glycin-N,N-Diessigsäure oder Derivate der Glutamin-N,N-Diessigsäure. Bevorzugt sind auch Derivate der Ethylendiamintriessigsäure oder der Nitrilotriessigsäure.

Besonders bevorzugt sind als Derivate der Glycin-N,N-Diessigsäure Alkalisalze der Methylglycin-N,N-diessigsäure, im Folgenden als MGDA bezeichnet.

Die wässrige Ausgangslösung wird in den Treibstrahl eines Strahlapparates eingesaugt, vom Treibstrahl verdüst und getrocknet, unter Erhalt des Granulates, das im oberen Bereich des Strahlapparates ausgetragen wird.

Entsprechend der Begriffbestimmung in der oben in der Beschreibungseinleitung angegebenen Diplomarbeit von A. Werner, handelt es sich bei der Wirbelschicht-Sprühgranulation um ein Formgebendes Verfahren um grobkörnige, monodisperse und nahezu runde Partikel zu erzeugen, mit Partikelgrößen im Bereich zwischen 0,3 mm und 30 mm, die vorliegend als Granulate bezeichnet werden, ausgehend von Lösungen, Suspensionen oder Schmelzen.

Die Wrbelschicht-Sprühgranulation wird in einer so genannten Strahlschicht durchgeführt, die eine spezielle Variante einer Wirbelschicht ist: In einer Standard-Wirbelschicht werden Partikel fluidisiert, indem über einen perforierten Boden, mit einer Vielzahl von Perforationen (Öffnungen) von unten Gas in ein Partikel kollektiv eingeblasen wird, wobei die Partikel aufgewirbelt werden.

Demgegenüber erfolgt bei einer so genannten Strahlschicht die Fluidisierung über eine oder mehrere wenige Öffnungen am Boden des Strahlapparates, über die ein oder mehrere Treibstrahle eingedüst werden. Der eine oder die mehreren Treibstrahle induzieren eine interne, geordnete Schlaufenbewegung, die auch als Kreislaufbewegung oder walzenförmige Bewegung bezeichnet werden kann, und die im Wesentlichen drei Fluidisationszustände oder -zonen umfasst, und zwar eine Strahlzone, eine Fontänenzone und eine Rücklaufzone. In einer ersten Zone oder Strahlzone erfolgt die Beschleunigung der Feststoffpartikeln unter Einwirkung des definiert, von unten nach oben geführten Treibstrahles, wobei sich die Partikeln in dieser Zone in der Strömungsrichtung des Treibstrahles bewegen. Entsprechend herrscht in der Strahlzone eine vertikal nach oben gerichtete Strömung vor. In einer anschließenden zweiten Zone oder Fontänenzone ändern die Feststoffpartikeln ihre Strömungsrichtung, es herrscht eine Querströmung vor. Schließlich gelangen die Partikeln in eine dritte Zone oder Rücklaufzone im Wandbereich des Strahlapparates und weisen dort eine nach unten gerichtete Bewegung auf, bis sie erneut in den Bereich des, von unten nach oben geführten Treibstrahles gelangen und in der ersten Zone, der Strahlzone, erneut vom Treibstrahl mitgerissen werden. In der Rücklaufzone bewegen sich die Partikeln typischerweise unter dem Einfluss der Schwerkraft.

Als Strahlapparat kann bevorzugt ein zylindrischer Apparat eingesetzt werden, der sich im unteren Bereich desselben kegelförmig verjüngt, indem von unten ein zentraler Treibstrahl eingedüst wird.

In einer weiteren bevorzugten Ausführungsform kann ein Treibstrahlapparat mit rechteckigem Querschnitt eingesetzt werden, der sich in einem unteren Teil verjüngt, und in dem im Bereich der Mittelachse desselben ein oder mehrere Treibstrahle von unten nach oben positioniert sind. Ein derartiger Apparat ist beispielsweise in DE 103 22 062 beschrieben.

Bevorzugt werden der eine oder die mehreren gasförmigen Treibstrahle aus einem Gas gebildet, das unter einem Überdruck im Bereich von 20 mbar bis 1 bar oberhalb des Druckes im Strahlapparat steht, das sich über eine Öffnung in den Strahlapparat entspannt und dabei den gasförmigen Treibstrahl bildet. Der Treibstrahl ist aus einem Gasstrom gebildet, der bevorzugt ein Inertgas, insbesondere Luft ist. In einer besonders bevorzugten Ausführungsform des vorliegenden Verfahrens wird in den Strahlapparat getrennt von der wässrigen Ausgangslösung, kristalliner Feinstaub mit einem mittleren Partikeldurchmesser im Bereich von etwa 1 bis 100 µm, bevorzugt von etwa 1 bis 20 µm, an einer Stelle in den Strahlapparat zugegeben, die verschieden ist von der Stelle, an der die wässrige Ausgangslösung eingedüst wird.

In einer vorteilhaften Ausführungsform enthält der kristalline Feinstaub dasselbe oder dieselben Komplexbildnersalze, wie sie in der wässrigen Ausgangslösung enthalten sind, oder eines oder mehrere hiervon verschiedene Komplexbildnersalze.

In einer weiteren bevorzugten Ausführungsform wird die wässrige Ausgangslösung mit kristallinem Feinstaub mit einem mittleren Partikeldurchmesser im Bereich von etwa 1 bis 100 µm, bevorzugt von etwa 1 bis 20 µm unter Erhalt einer Suspension vorvermischt, im unteren Bereich des Strahlapparates eingedüst und vom Treibstrahl eingesaugt.

Es ist auch möglich, kaskadierte Strahlapparate einzusetzen, das heißt mehrere hintereinander geschaltete, wie oben beschrieben ausgestaltete Apparate.

Bevorzugt hat der den Treibstrahl bildende Gasstrom eine Temperatur im Bereich zwischen 80 und 450°C, weiter bevorzugt zwischen 120 und 240°C.

Die Temperatur in der Strahlzone, in der Fontänenzone und in der Rücklaufzone liegt bevorzugt im Bereich zwischen 70 und 150 °C.

Die Verweilzeit im Strahlapparat liegt bevorzugt zwischen 1 Minute und 1 Stunde, insbesondere zwischen 10 Minuten und 40 Minuten.

Gegenstand der Erfindung sind auch Formulierungen, enthaltend das nach dem vorstehend beschriebenen Verfahren hergestellte Granulat oder wässrige Lösungen hiervon als Komplexbildner für Erdalkali- und Schwermetallionen, in den hierfür üblichen Mengen, neben anderen üblichen Bestandteilen solcher Formulierungen.

Bevorzugt handelt es sich dabei um Wasch- und Reinigungsmittelformulierungen handelt.

Gegenstand der Erfindung ist auch die Verwendung des nach dem vorstehend beschriebenen Verfahren hergestellten Granulates zur Herstellung von Pressagglomeraten.

Bevorzugt werden die Pressagglomerate in festen Reinigungsmitteln eingesetzt, die insbesondere zum Einsatz in Geschirrspülmaschinen bestimmt sind.

Die Erfindung wird im Folgenden anhand von Ausführungsbeispielen näher erläutert.

### Beispiel erfindungsgemäß)

In einem Strahlapparat vom Typ ProCell 5 der Fa. Glatt, wurde eine 40%ige wässrige Trilon M^{®} Lösung (Trinatriumsalz der Methylglycin-N,N-diessigsäure) sprühgranuliert. Der Strahlapparat wurde mit einem Volumenstrom von 180 Nm³/h Luft einer Eintrittstemperatur von 180°C als Treibstrahl betrieben. In den unteren Bereich des Strahlapparates wurden 9,5 kg/h der wässrigen Lösung mittels einer Zweistoffdüse eingesprüht. Als Zerstäubungsgas wurde dabei Druckluft mit 8 bar eingesetzt. Die Temperatur im Strahlapparat betrug 80°C.

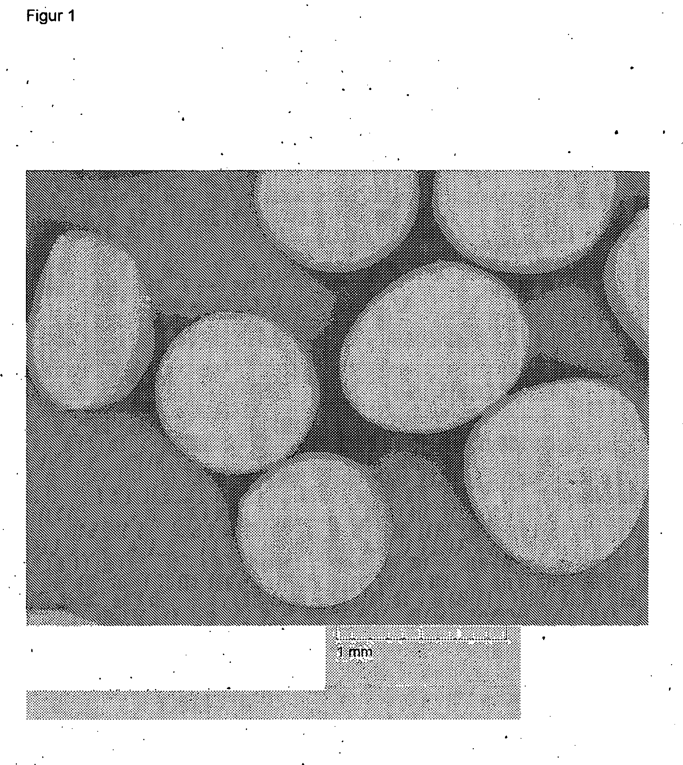

Unter diesen Bedingungen wurde ein rundes, kompaktes Granulat entsprechend den in Figur 1 dargestellten REM-Aufnahmen erhalten. Die Partikelgröße lag im Bereich von 0,4 bis 2 mm und das Schüttgewicht betrug 900 kg/m³.

### Vergleichsbeispiel

Zum Vergleich wurde die gleiche, 40%ige wässrige Trilon M^{®} Lösung sprühgranuliert, jedoch in einer Wirbelschicht. Hierfür wurde ein Wirbelschichtapparat mit einem Durchmesser von 300 mm der BASF SE eingesetzt.

Die Granulierwirbelschicht wurde mit einem Volumenstrom von 205 Nm³/h Luft einer Eintrittstemperatur von 160°C betrieben. In den unteren Bereich der Granulierwirbelschicht wurden 5,3 kg/h der wässrigen Lösung mit einer Zweistoffdüse eingesprüht. Als Zerstäubungsgas wurde dabei Druckluft mit 5 bar eingesetzt. Die Temperatur in der Granulierwirbelschicht betrug 100°C.

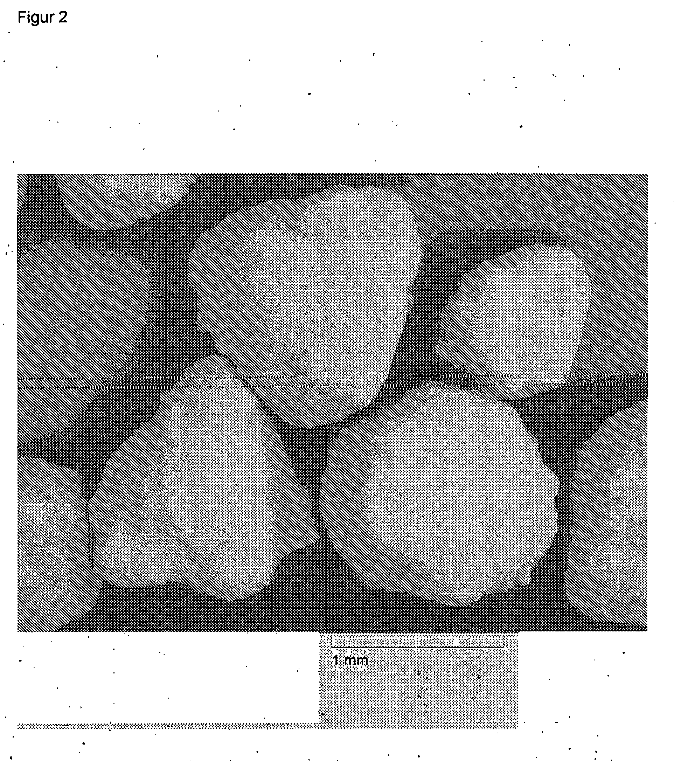

Unter diesen Bedingungen wurde ein unregelmäßig geformtes Granulat entsprechend der in Figur 2 dargestellten REM-Aufnahmen erhalten. Die Partikelgröße lag im Bereich von 0.4 bis 2 mm und das Schüttgewicht betrug 750 kg/m³.

Die Versuche belegen somit, dass nach dem erfindungsgemäßen Verfahren eine wesentlich gleichmäßigere Partikelform und ein deutlich erhöhtes Schüttgewicht des Granulats erreicht werden.

## Patentansprüche

1. Verfahren zur Herstellung eines Granulates enthaltend eines oder mehrere Komplexbildnersalze der allgemeinen Formel 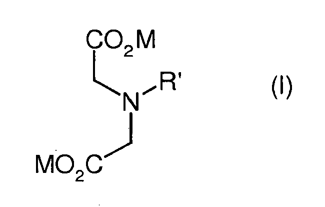 in der
R' Wasserstoff oder eine der Gruppierungen 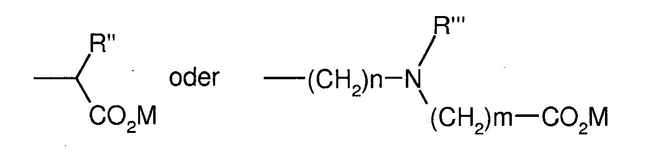 bedeutet, wobei
R'' Wasserstoff, ein C₁-C₁₂-Alkylrest oder ein -(CH₂)_{q}-COOM-Rest mit q = 1 bis 5 ist
n und m jeweils eine ganze Zahl von 0 bis 5 sind und
R''' Wasserstoff oder ein C₁-C₁₂-Alkylrest oder ein C₂-C₁₂-Alkenylrest ist, der zusätzlich mit bis zu 5 Hydroxylgruppen substituiert sein kann, oder eine der Gruppierungen 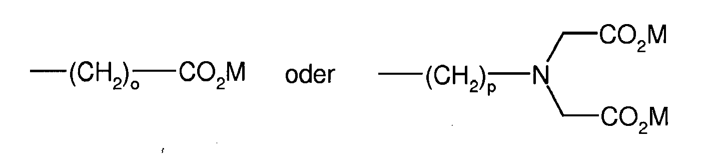
ist, in der o und p jeweils eine ganze Zahl von 0 bis 5 sind, und
M unabhängig voneinander Wasserstoff, Alkalimetall, Erdalkalimetall, Ammonium oder substituiertes Ammonium in den entsprechenden stöchiometrischen Mengen bedeutet,
- aus einer wässrigen Ausgangslösung, enthaltend das eine oder die mehreren Komplexbildnersalze in einer Konzentration von 10 bis 80 Gew.-%, bezogen auf das Gesamtgewicht der wässrigen Ausgangslösung,
in einem Strahlapparat, in dem ein von unten nach oben gerichteter, zentral oder ein oder mehrere, im Bereich der Mittelachse des Strahlapparates positionierte gasförmige Treibstrahle eine interne Schlaufenbewegung induzieren, unter Ausbildung einer Strahlzone, an deren oberen Ende sich eine Fontänenzone anschließt, die in eine Rücklaufzone im Wandbereich des Strahlapparates übergeht, die erneut, in ihrem unteren Bereich, in die Strahlzone übergeht, wo die wässrige Ausgangslösung in den einen oder die mehreren gasförmigen Treibstrahle eingedüst wird und dabei unter Erhalt des Granulates getrocknet wird, das aus dem Strahlapparat ausgetragen wird.

2. Verfahren nach Anspruch 1, **dadurch gekennzeichnet, dass** der Strahlapparat ein zylindrischer Apparat ist, der sich im unteren Bereich kegelförmig verjüngt, und in den ein zentraler gasförmiger Treibstrahl eingedüst wird.

3. Verfahren nach Anspruch 1, **dadurch gekennzeichnet, dass** der Strahlapparat einen rechteckigen Querschnitt aufweist, der sich in seinem unteren Teil verjüngt, und der ein oder mehrere, im Bereich der Mittelachse des Strahlapparates positionierte, von unten nach oben gerichtete gasförmige Treibstrahle aufweist.

4. Verfahren nach einem der Ansprüche 1 bis 3, **dadurch gekennzeichnet, dass** der eine oder die mehreren gasförmigen Treibstrahle aus einem Gas gebildet sind, das unter einem Überdruck im Bereich von 20 mbar bis 1 bar oberhalb des Druckes im Strahlapparat steht, das sich über eine Öffnung in den Strahlapparat entspannt und dabei den gasförmigen Treibstrahl bildet.

5. Verfahren nach einem der Ansprüche 1 bis 4, **dadurch gekennzeichnet, dass** über eine oder mehrere Ein- oder Zweistoffdüsen in den unteren Bereich des Strahlapparates die wässrige Ausgangslösung eingedüst wird.

6. Verfahren nach einem der Ansprüche 1 bis 5, **dadurch gekennzeichnet, dass** in den Strahlapparat, getrennt von der wässrigen Ausgangslösung, kristalliner Feinstaub mit einem mittleren Partikeldurchmesser im Bereich von etwa 1 bis 100 µm, bevorzugt von etwa 1 bis 20 µm, an einer Stelle in den Strahlapparat zugegeben wird, die verschieden ist von der Stelle, an der die wässrige Ausgangslösung eingedüst wird.

7. Verfahren nach Anspruch 6, **dadurch gekennzeichnet, dass** der kristalline Feinstaub dasselbe oder dieselben Komplexbildnersalze, wie sie in der wässrigen Ausgangslösung enthalten sind, oder eines oder mehrere hiervon verschiedene Komplexbildnersalze enthält.

8. Verfahren nach einem der Ansprüche 1 bis 5, **dadurch gekennzeichnet, dass** die wässrige Ausgangslösung und kristalliner Feinstaub mit einem mittleren Partikeldurchmesser im Bereich von etwa 1 bis 100 µm, bevorzugt von etwa 1 bis 20 µm unter Erhalt einer Suspension vorvermischt, im unteren Bereich des Strahlapparates eingedüst und vom Treibstrahl eingesaugt werden.

9. Verfahren nach einem der Ansprüche 1 bis 8, **dadurch gekennzeichnet, dass** der den Treibstrahl bildende Gasstrom eine Temperatur im Bereich zwischen 80 und 450°C, bevorzugt zwischen 120 und 240°C, hat.

10. Verfahren nach einem der Ansprüche 1 bis 9, **dadurch gekennzeichnet, dass** die Temperatur in der Strahlzone, in der Fontänenzone und in der Rücklaufzone im Bereich zwischen 70 und 150 °C liegt.

11. Verfahren nach einem der Ansprüche 1 bis 10, **dadurch gekennzeichnet, dass** die mittlere Verweilzeit des Granulates im Strahlapparat zwischen 1 Minute und 1 Stunde, bevorzugt zwischen 10 Minuten und 40 Minuten, liegt.

## Claims

1. A process for producing granules comprising one or more complexing agent salts of the general formula 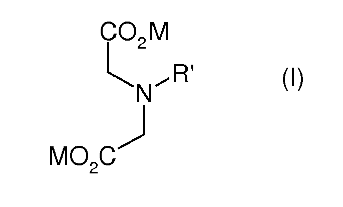 in which
R' is hydrogen or one of the groups 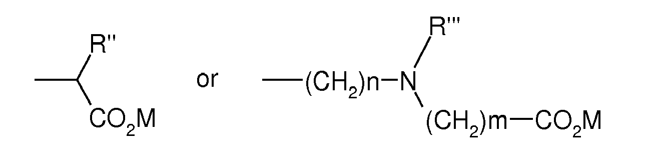 where
R'' is hydrogen, a C₁-C₁₂-alkyl radical or a -(CH₂)_{q}-COOM radical where q = 1 to 5
n and m are in each case an integer from 0 to 5 and
R''' is hydrogen or a C₁-C₁₂-alkyl radical or a C₂-C₁₂-alkenyl radical, which may be additionally substituted with up to 5 hydroxyl groups, or is one of the groups 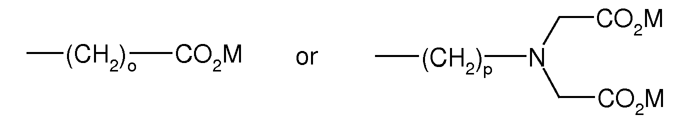 in which o and p are in each case an integer from 0 to 5, and
M, independently of the others, is hydrogen, alkali metal, alkaline earth metal, ammonium or substituted ammonium in the corresponding stoichiometric amounts,
- from an aqueous starting solution comprising the one or more complexing agent salts in a concentration of from 10 to 80% by weight, based on the total weight of the aqueous starting solution, in a jet apparatus, in which a gaseous driving jet positioned centrally and directed from bottom to top, or one or more gaseous driving jets positioned in the region of the central axis of the jet apparatus induce an internal loop motion, with formation of a jet zone, joined to the upper end of which is a fountain zone, which merges into a return zone in the wall region of the jet apparatus, which return zone in turn, in its lower region, merges into the jet zone, where the aqueous starting solution is sprayed into the one or more gaseous driving jets and in so doing is dried to give the granules, which are discharged from the jet apparatus.

2. The process according to claim 1, wherein the jet apparatus is a cylindrical apparatus which is conically tapered in the lower region, and into which a central gaseous driving jet is sprayed.

3. The process according to claim 1, wherein the jet apparatus has a rectangular cross section which tapers in its lower section and which has one or more gaseous driving jets positioned in the region of the central axis of the jet apparatus and directed from bottom to top.

4. The process according to any one of claims 1 to 3, wherein the one or more gaseous driving jets are formed from a gas which is under a positive pressure in the range from 20 mbar to 1 bar above the pressure in the jet apparatus which decompresses via an opening in the jet apparatus and in so doing forms the gaseous driving jet.

5. The process according to any one of claims 1 to 4, wherein the aqueous starting solution is sprayed in via one or more single-material or two-material nozzles into the lower region of the jet apparatus.

6. The process according to any one of claims 1 to 5, wherein crystalline fine dust with an average particle diameter in the range from about 1 to 100 µm, preferably from about 1 to 20 µm, is added to the jet apparatus, separately from the aqueous starting solution, at a position in the jet apparatus which is different from the position at which the aqueous starting solution is sprayed in.

7. The process according to claim 6, wherein the crystalline fine dust comprises the same complexing agent salt(s) as are present in the aqueous starting solution, or one or more complexing agent salts different therefrom.

8. The process according to any one of claims 1 to 5, wherein the aqueous starting solution and crystalline fine dust with an average particle diameter in the range from about 1 to 100 µm, preferably from about 1 to 20 µm are premixed to give a suspension, sprayed in in the lower region of the jet apparatus and sucked in by the driving jet.

9. The process according to any one of claims 1 to 8, wherein the gas stream forming the driving jet has a temperature in the range between 80 and 450°C, preferably between 120 and 240°C.

10. The process according to any one of claims 1 to 9, wherein the temperature in the jet zone, in the fountain zone and in the return zone is in the range between 70 and 150°C.

11. The process according to any one of claims 1 to 10, wherein the average residence time of the granules in the jet apparatus is between 1 minute and 1 hour, preferably between 10 minutes and 40 minutes.

## Revendications

1. Procédé pour la préparation d'un granulat contenant un ou plusieurs sel(s) complexant(s) de formule générale 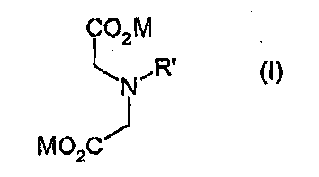 dans laquelle
R' signifie hydrogène ou un des groupes 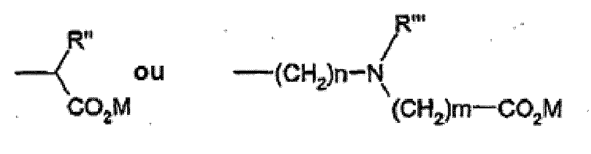 où
R'' représente hydrogène, un radical C₁-C₁₂-alkyle ou un radical -(CH₂)_{q}-COOM avec q = 1 à 5
n et m signifient à chaque fois un nombre entier de 0 à 5 et
R''' représente hydrogène ou un radical C₁-C₁₂-alkyle ou un radical C₂-C₁₂-alcényle, qui peut en outre être substitué par jusqu'à 5 groupes hydroxyle, ou un des groupes 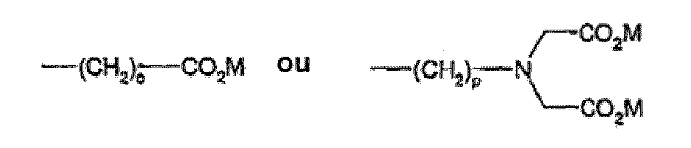
dans lesquels o et p signifient à chaque fois un nombre entier de 0 à 5 et
M signifie, indépendamment les uns des autres, hydrogène, métal alcalin, métal alcalino-terreux, ammonium ou ammonium substitué aux quantités stoechiométriques correspondantes
- à partir d'une solution aqueuse de départ, contenant ledit un ou lesdits plusieurs sel(s) complexant(s) en une concentration de 10 à 80% en poids, par rapport au poids total de la solution aqueuse de départ,
dans un appareil à jets, dans lequel un jet propulseur gazeux et orienté de bas en haut, central ou un ou plusieurs jets propulseurs gazeux positionnés au niveau de l'axe central de l'appareil à jets indui(sen)t un mouvement interne en boucle, avec formation d'une zone à jets, suivie en son extrémité supérieure d'une zone à fontaine, qui devient une zone de reflux dans la partie de paroi de l'appareil à jets, qui devient à son tour, dans sa partie inférieure, la zone à jets, la solution aqueuse de départ étant injectée dans ledit un ou lesdits plusieurs jet(s) propulseur(s) gazeux et y étant séchée avec obtention du granulat qui est évacué de l'appareil à jets.

2. Procédé selon la revendication 1, **caractérisé en ce que** l'appareil à jets est un appareil cylindrique, qui se rétrécit en forme de cône dans la partie inférieure et dans lequel un jet propulseur gazeux central est injecté.

3. Procédé selon la revendication 1, **caractérisé en ce que** l'appareil à jets présente une section transversale rectangulaire, qui se rétrécit dans sa partie inférieure et qui présente un ou plusieurs jet(s) propulseur(s) gazeux positionné(s) au niveau de l'axe central de l'appareil à jets, orienté(s) de bas en haut.

4. Procédé selon l'une quelconque des revendications 1 à 3, **caractérisé en ce que** ledit un ou lesdits plusieurs jet(s) propulseur(s) gazeux est/sont formé(s) à partir d'un gaz qui se trouve sous une surpression dans la plage de 20 mbars à 1 bar au-dessus de la pression dans l'appareil à jets, qui se détend via une ouverture dans l'appareil à jets et qui forme ainsi le jet propulseur gazeux.

5. Procédé selon l'une quelconque des revendications 1 à 4, **caractérisé en ce que** la solution aqueuse de départ est injectée via un ou plusieurs gicleur (s) à une ou deux substance (s) dans la partie inférieure de l'appareil à jets.

6. Procédé selon l'une quelconque des revendications 1 à 5, **caractérisé en ce que** de la poussière fine cristalline, présentant un diamètre de particule moyen dans la plage d'environ 1 à 100 µm, de préférence d'environ 1 à 20 µm, est ajoutée dans l'appareil à jets, séparément de la solution aqueuse de départ, en un endroit dans l'appareil à jets qui est différent de l'endroit où la solution aqueuse de départ est injectée.

7. Procédé selon la revendication 6, **caractérisé en ce que** la poussière fine cristalline contient le même ou les mêmes sel(s) complexant(s) que celui/ceux qui est/sont contenu(s) dans la solution aqueuse de départ ou un ou plusieurs sel(s) complexant(s) différent(s) de celui/ceux-ci.

8. Procédé selon l'une quelconque des revendications 1 à 5, **caractérisé en ce que** la solution aqueuse de départ et la poussière fine cristalline présentant un diamètre de particule moyen dans la plage d'environ 1 à 100 µm, de préférence d'environ 1 à 20 µm, sont prémélangées avec obtention d'une suspension, injectées dans la partie inférieure de l'appareil à jets et aspirées par le jet propulseur.

9. Procédé selon l'une quelconque des revendications 1 à 8, **caractérisé en ce que** le flux gazeux formant le jet propulseur présente une température dans la plage entre 80 et 450°C, de préférence entre 120 et 240°C.

10. Procédé selon l'une quelconque des revendications 1 à 9, **caractérisé en ce que** la température, dans la zone à jets, dans la zone à fontaine et dans la zone de reflux, se situe dans la plage entre 70°C et 150°C.

11. Procédé selon l'une quelconque des revendications 1 à 10, **caractérisé en ce que** la durée de séjour moyenne du granulat dans l'appareil à jets se situe entre 1 minute et 1 heure, de préférence entre 10 minutes et 40 minutes.
